# EUROPEAN PATENT SPECIFICATION

(11) **EP 4 324 491 B1**
(45) Date of publication and mention of the grant of the patent: **28.10.2026**
(21) Application number: 22933096.4
(22) Date of filing: 10.11.2022
(51) Int. Cl.: A61L 27/20, A61L 27/36, A61L 27/58, D04H 1/728

(54) **BIONIC NERVE GRAFT AND PREPARATION METHOD THEREFOR**
BIONISCHES NERVENTRANSPLANTAT UND HERSTELLUNGSVERFAHREN DAFÜR
GREFFE DE NERF BIONIQUE ET SA MÉTHODE DE PRÉPARATION

(30) Priority: 18.10.2022 CN 202211269678
(43) Date of publication of application: 21.02.2024
(73) Proprietor: Nantong University, Nantong, Jiangsu 226019 (CN)
(72) Inventor: GU, Xiaosong, Nantong, Jiangsu 226019 (CN); DAI, Xiu, Nantong, Jiangsu 226019 (CN); GU, Xingzhong, Nantong, Jiangsu 226019 (CN); GONG, Leilei, Nantong, Jiangsu 226019 (CN); LI, Meiyuan, Nantong, Jiangsu 226019 (CN); SUN, Hualin, Nantong, Jiangsu 226019 (CN); QIAN, Tianmei, Nantong, Jiangsu 226019 (CN); WANG, Hongkui, Nantong, Jiangsu 226019 (CN); XU, Lai, Nantong, Jiangsu 226019 (CN)
(74) Representative: Proi World Intellectual Property GmbH
(86) International application number: PCT/CN2022/131041
(87) International publication number: WO 2023/179045

(56) References cited:
- CN-A- 1 385 217
- CN-A- 1 580 255
- CN-A- 101 433 475
- CN-A- 109 701 075
- US-A1- 2014 379 009
- US-A1- 2017 172 578
- US-A1- 2019 328 393

## Description

### BACKGROUND OF THE INVENTION

### 1. Technical Field

The present invention belongs to the technical field of biological materials, and in particular, relates to a bionic nerve graft and a preparation method thereof.

### 2. Description of Related Art

Peripheral nerve defect is a common clinical disease with poor prognosis and high disability rate, which seriously affects the quality of life of patients. At present, autotransplantation is still the "gold standard" for repairing the peripheral nerve defect. However, the clinical application of autotransplantation is limited by the problems of limited source of nerve donors, unmatched size of donor nerves and receptor nerves, and wrong nerve function bundles.

A tissue engineering scaffold material has the advantages of adjustable structural size, abundant material source and high processibility, and provides a new treatment method for the recovery of injured nerves. With the progress of a micro-nano manufacturing technology such as electrospinning, microetching, 3D printing and separation, bionic tissue engineering nerve grafts with porous structures, ordered structures, multi-channel structures, multi-layer structures or multi-branch structures have been constructed. In recent 20 years, in the construction process of the nerve graft, a large amount of research work shows that nerve tissue engineering scaffolds with a directional microstructure can induce the directional arrangement and growth of nerve cells (Rao Feng. Aligned chitosan nanofiber hydrogel grafted with peptides mimicking bioactive brain-derived neurotrophic factor and vascular endothelial growth factor repair long-distance sciatic nerve defects in rats. Theranostics, 2020, 10(4): 1590-1603). Although researchers have made some progress in the construction of the bionic tissue engineering nerve graft in recent years, the reconstruction of the microenvironment still has some challenges. No standard reconstruction scheme can achieve the perfect balance between the microenvironment stability and the functional reconstruction of the bionic nerve graft.

A natural biological material has the advantages of high biocompatibility, easy absorption and capability of promoting cell adhesion and proliferation, and has been widely applied to the preparation of a nerve regeneration graft. However, the natural biological material has poor mechanical property, which is easy to cause breakage and collapse, thereby greatly affecting the microenvironment stability of the regenerated nerves. Good mechanical property is necessary to maintain the microenvironment stability. To provide a sufficient mechanical support for the reconstruction of the regenerated nerve microenvironment, the mechanical property of the nerve graft can be improved by a method for adding a crosslinking agent or a synthetic macromolecule with excellent mechanical property. However, the crosslinking agent, particularly a chemical crosslinking agent, has high toxicity and is difficult to remove, and the biocompatibility of the synthetic macromolecule such as polycaprolactone (PCL) or polylactic acid (PLA) is far less than that of the natural material, so the crosslinking agent and the synthetic macromolecule are not conducive to the microenvironment reconstruction and the recovery of the nerve function. How to prepare the nerve graft with excellent mechanical property and excellent biocompatibility is a problem to be solved in the construction process of the bionic nerve graft.

CN 1580255 A discloses a tissue engineered peripheral nerve graft, comprising a stent for connecting two broken ends of a nerve and seed cells having a function of promoting nerve regeneration.

### BRIEF SUMMARY OF THE INVENTION

An objective of the present invention is to overcome the defects in the prior art and provide a bionic nerve graft and a preparation method thereof. The method is simple and low in cost. The prepared bionic nerve graft ensures sufficient mechanical support, provides a sufficient space for the transmission of nerve regeneration nutrients, and has high biocompatibility and low immunogenicity.

The present invention provides the following technical solutions:
according to a first aspect, a bionic nerve graft is provided and includes a helical stent, a nanofiber membrane, guide fibers and a human bone marrow stromal cell-extracellular matrix (hBMSC-ECM), where a surface of the helical stent is coated with the nanofiber membrane; the guide fibers and the hBMSC-ECM are placed in a tube cavity of the helical stent; and the raw materials of the helical stent, the nanofiber membrane and the guide fibers are all chitosan.

Further, the helical stent has an inner diameter of 1-6 mm, a length of 1-6 cm and a pitch of 0.5-5 mm.

Further, the guide fibers have a length of 1-3 cm, and the number of the guide fibers is 3-28.

According to a second aspect, a preparation method of the bionic nerve graft according to the first aspect is provided and includes the following steps:
using the chitosan as a raw material and preparing the helical stent by a mold pouring method;
using the chitosan as a raw material and coating the surface of the helical stent with one layer of nanofiber membrane to obtain a helical tube with a surface coated with the nanofiber membrane;
using the chitosan as a raw material and preparing the guide fibers by a template method; and
filling the guide fibers and the hBMSC-ECM in the helical tube with the surface coated with the nanofiber membrane to obtain the bionic nerve graft.

Further, the step of preparing the helical stent includes:
preparing a chitosan solution with a concentration of 4wt%-7wt%;
pouring the chitosan solution on a threaded inner core mold;
placing the inner core mold with the chitosan solution into a NaOH solution of 5-20% for curing and shaping, and then performing washing with water, drying and demolding to obtain a helical stent semi-finished product; and
immersing the helical stent semi-finished product into an acetic anhydride solution containing methanol of 5wt% to perform acetylation for 6 h, performing fixation for 12 h by using the NaOH solution of 5-20%, and washing and drying to obtain the helical stent.

Further, the step of preparing the helical tube with the surface coated with the nanofiber membrane includes:
preparing a blended solution of the chitosan and polyethylene oxide with the total concentration of 3wt%-5wt%, the mass ratio of the chitosan to the polyethylene oxide being 100:(5-20);
placing the blended solution of the chitosan and the polyethylene oxide into an electrostatic spinning apparatus, placing the helical stent on a tubular receiving apparatus, performing electrostatic spinning, and coating the surface of the helical stent with one layer of nanofiber membrane; and
placing the helical stent coated with the nanofiber membrane into a NaOH solution of 5-20% for curing and shaping, performing washing with water to remove the polyethylene oxide to obtain the helical tube with the surface coated with the nanofiber membrane.

Further, setting parameters of the electrostatic spinning include: a voltage is 5-35 kV, a flow velocity is 0.1-2 mL/h, a receiving distance is 5-35 cm, and a rotating speed of the receiving apparatus is 5-150 rpm.

Further, the step of preparing the guide fibers includes:
preparing a chitosan solution with a concentration of 4wt%-7wt%;
pouring the chitosan solution in a mold with a groove; and
putting the mold with the chitosan solution into a NaOH solution of 5-20% for curing and shaping, and then performing washing with water, drying and demolding to obtain the guide fibers.

Further, the groove of the mold includes an inner groove and an outer groove which communicate with each other, the cross-sectional shape of the communicated inner groove and outer groove is shaped like an inverted Chinese character "Pin", a width of the outer groove is 100-500 µm, a height of the outer groove is 20-50 µm, a width of the inner groove is the same as the height of the outer groove, a height of the inner groove is 25-240 µm, the width of the outer groove is twice the sum of the height of the inner groove and the width of the inner groove, and the inner groove and the outer groove have the same length of 1-3 cm.

Further, the step of preparing the hBMSC-ECM includes:
coating a culture dish with a mesenchymal stem cell (MSC) adherent reagent diluted 100 times by a DPBS solution;
planting resuscitated human bone marrow stromal cell (hBMSC) into the coated culture dish, adding a complete culture medium, performing culture in a 5% CO₂ incubator at 37°C, and changing the culture medium once every two days; and
when the cell saturation reaches 80%, using a complete culture medium containing ascorbic acid (50µg/mL), changing the solution once every two days in a dark place, discarding the culture solution after ten days, performing washing with DPBS for three times, performing hypotonic operation with ultrapure water at 37°C for 10 min, removing the ultrapure water, adding a cell lysis solution to perform reaction for 5 min, performing washing with DPBS, performing reaction by using DNA enzyme (0.1mg/mL) at 37°C for 2 h after washing, and finally, performing washing with DPBS and performing storage at 4°C.

Compared with the prior art, the present invention has the following beneficial effects:
(1) the bionic nerve graft provided by the present invention includes a helical stent, a nanofiber membrane, guide fibers and hBMSC-ECM, where a surface of the helical stent is coated by the nanofiber membrane, thereby ensuring that the bionic nerve graft has sufficient mechanical support and good compliance and provides a sufficient space for the transmission of nerve regeneration nutrients; the guide fibers and the hBMSC-ECM are placed in a tube cavity of the helical stent to play a good induction role, thereby facilitating the directional arrangement and growth of nerve cells; and nerve regeneration microenvironment elements are provided, thereby facilitating the regeneration of injured nerves.
(2) In the bionic nerve graft provided by the present invention, the raw materials of the helical stent, the nanofiber membrane and the guide fibers are all chitosan; the chitosan is a degradable natural material, has the advantages of high biocompatibility and low immunogenicity, and is conducive to the automatic step-by-step degradation of the nerve graft in the nerve repairing process; and the degradation product is completely absorbed by a human body, thereby avoiding immune tissue reaction.
(3) In the preparation method of the bionic nerve graft provided by the present invention, the helical stent is prepared by a mold pouring method, the surface of the helical stent is coated with one layer of nanofiber membrane by an electrostatic spinning method, and the mold pouring technology and the electrostatic spinning technology are combined, so that the bionic nerve graft can realize mechanical support and the transmission of the nerve regeneration nutrients at the same time, the total mass of the bionic nerve graft can be reduced, and the immunological rejection is greatly reduced.
(4) In the preparation method of the bionic nerve graft provided by the present invention, the technologies such as molding pouring, the template method and electrostatic spinning are simple and practicable, the cost is low, and the production process is safe and controllable.

### BRIEF DESCRIPTION OF THE SEVERAL VIEWS OF THE DRAWINGS

FIG. 1 is a scanning electron microscope (SEM) diagram of a nanofiber membrane in Embodiment 1;
FIG. 2 is an SEM diagram of a helical tube with a surface coated with a nanofiber membrane in Embodiment 1;
FIG. 3 is a schematic diagram of a sectional view of a mold groove for preparing guide fibers in an embodiment;
FIG. 4 is an SEM diagram of guide fibers in Embodiment 2;
FIG. 5 is DNA content of hBMSCs-ECM before and after decellularization in Embodiment 2;
FIG. 6 is an HE staining diagram of 6-week in-vivo degradation of an acetylated helical stent in Embodiment 2;
FIG. 7 is an immunofluorescence picture of a transverse section of a distal end of a regenerated nerve and an axon density of the distal end of the regenerated nerve obtained 8 weeks after a nerve graft in Embodiment 2 repairs a 10mm sciatic nerve space of a rat (TEN: bionic nerve group, AUTO: autogenous nerve group, SHAM: sham-operation group, the same below);
FIG. 8 is a transmission electron microscope (scale: left 50 µm, right 0.2 µm) of three groups of animal regenerated nerves obtained 12 weeks after operation in Embodiment 2, and a histogram of myelin sheath layers and myelin sheath thickness among the three groups;
FIG. 9 is a composite muscle action potential diagram, conduction velocity, proximal amplitude and distal amplitude diagrams obtained 12 weeks after operation in Embodiment 2; and
FIG. 10 is a CatWalk gait analysis diagram 12 weeks after operation in Embodiment 2.

### DETAILED DESCRIPTION OF THE INVENTION

The present invention is further described below with reference to the accompanying drawings. The following embodiments are only used for describing the technical solutions of the present invention more clearly, and are not intended to limit the protection scope of the present invention.

### Embodiment 1

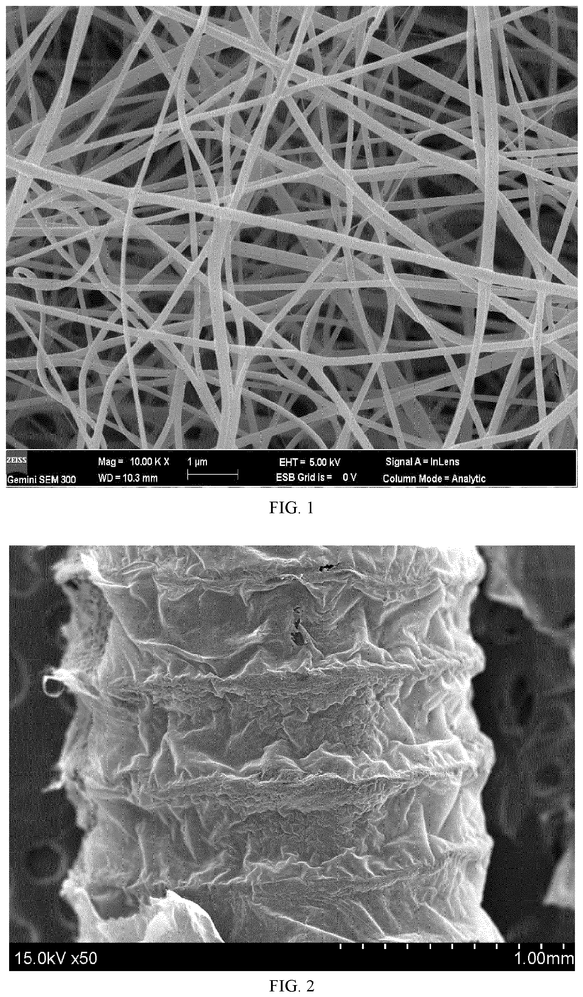

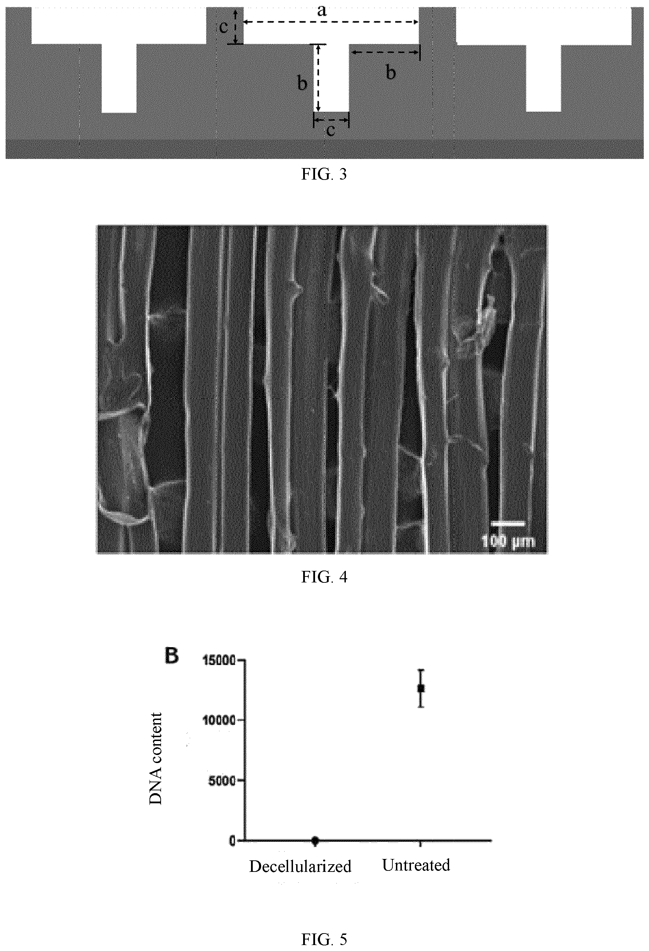

(1) Chitosan was dissolved in an acetic acid solution of 2% and was stirred for 30 min until being completely dissolved to obtain a chitosan solution with the concentration of 4wt%; the chitosan solution was poured on a threaded inner core mold, where the inner mold has a length of 1 cm, a diameter of 2 mm, a thread pitch of 1 mm and a line width of 0.5 mm; the inner core mold with the chitosan solution was put into a NaOH solution of 5% for curing and shaping, was washed with water after being shaped, and was dried and demolded to obtain a helical stent semi-finished product; the helical stent semi-finished product was immersed into an acetic anhydride solution containing 5wt% methanol for acetylation for 6 h, then was fixed for 12 h at room temperature by a NaOH solution of 5%, and was washed and dried to obtain a helical stent.
(2) A proper amount of polyethylene oxide was dissolved into an acetic acid solution of 50%, and chitosan powder was added to prepare an electrostatic spinning solution, where the total concentration of the chitosan and the polyethylene oxide is 3wt% and the mass ratio of the chitosan to the polyethylene oxide is 100:20; the helical stent was placed in a rotatable tubular receiving apparatus, the electrostatic spinning solution was put into an electrostatic spinning machine injector, a voltage was set to 5 kV, a flow velocity was set to 0.1 ml/h, a receiving distance was set to 5 cm, a rotating speed of the receiving apparatus was set to 5 rpm, electrostatic spinning was performed, and a surface of the helical stent was coated with one layer of nanofiber membrane (as shown in FIG. 1) to obtain a helical tube with a surface coated with the nanofiber membrane (as shown in FIG. 2).
(3) The chitosan solution in step (1) was poured in a mold with a groove, where the groove includes an inner groove and an outer groove which communicate with each other, the cross-sectional shape is shown in FIG. 3, a length of the groove is 1 cm, a width a of the outer groove is 100 µm, a height c of the outer groove is 20 µm, the inner groove is located in the middle of the outer groove, a width of the inner groove is c, a height b of the inner groove is 40 µm, and the width of the outer groove is twice the sum of the height of the inner groove and the width of the inner groove. The mold with the chitosan solution was put into a NaOH solution of 10% for curing and shaping, was washed with water after being shaped, and was dried and demolded to obtain guide fibers.
(4) A culture dish was coated with an MSC adherent reagent diluted 100 times by a DPBS solution; resuscitated hBMSC was planted into the coated culture dish, a complete culture medium (a ratio of a NutriStem^{®} MSC XF basic culture medium to a NutriStem^{®} MSC XF additive is equal to 500ml:3ml) was added, culture was performed in a 5% CO₂ incubator at 37°C, and the culture medium was changed once every two days; and when the cell saturation reached about 80%, a complete culture medium containing ascorbic acid (50µg/mL) was used, the solution was changed once every two days in a dark place, the culture solution was discarded after ten days, washing was performed with DPBS for three times, hypotonic operation was performed with ultrapure water at 37°C for 10 min, the ultrapure water was removed, a cell lysis solution (3% TritonX-100, 2% lauryl sodium sulfate) was added to perform reaction for 5 min, washing was performed with DPBS, reaction was performed by using DNA enzyme (0.1mg/mL) at 37°C for 2 h after washing, and finally, washing was performed with DPBS for decellularization to obtain hBMSC-ECM.
(5) 28 guide fibers and the hBMSC-ECM were filled in the helical tube with the surface coated with the nanofiber membrane to obtain the bionic nerve graft, where the guide fibers are included in the tube wall of the helical stent.

### Embodiment 2

(1) Chitosan was dissolved in an acetic acid solution of 2% and was stirred for 2 h until being completely dissolved to obtain a chitosan solution with the concentration of 6wt%; the chitosan solution was poured on a threaded inner core mold, where the inner mold has a length of 4cm, a diameter of 2 mm, a thread pitch of 1 mm and a line width of 0.5 mm; the inner core mold with the chitosan solution was put into a NaOH solution of 5% for curing and shaping, was washed with water after being shaped, and was dried and demolded to obtain a helical stent semi-finished product; the helical stent semi-finished product was immersed into an acetic anhydride solution containing 5wt% methanol for acetylation for 6 h, then was fixed for 12 h at room temperature by a NaOH solution of 10%, and was washed and dried to obtain a helical stent.
(2) A proper amount of polyethylene oxide was dissolved into an acetic acid solution of 50%, and chitosan powder was added to prepare an electrostatic spinning solution, where the total concentration of the chitosan and the polyethylene oxide is 4wt% and the mass ratio of the chitosan to the polyethylene oxide is 100:20. The helical stent was placed in a rotatable tubular receiving apparatus, the electrostatic spinning solution was put into an electrostatic spinning machine injector, a voltage was set to 20 kV, a flow velocity was set to 0.1 ml/h, a receiving distance was set to 15 cm, a rotating speed of the receiving apparatus was set to 100 rpm, electrostatic spinning was performed, and a surface of the helical stent was coated with one layer of nanofiber membrane to obtain a helical tube with a surface coated with the nanofiber membrane.
(3) The chitosan solution in step (1) was poured in a mold with a groove, where the shape of the groove is the same as that in Embodiment 1, a length of the groove is 3 cm, a is 250 µm, b is 100 µm, and c s 50 µm; and the mold with the chitosan solution was put into a NaOH solution of 10% for curing and shaping, was washed with water after being shaped, and was dried and demolded to obtain guide fibers (as shown in FIG. 4).
(4) A culture dish was coated with an MSC adherent reagent diluted 100 times by a DPBS solution; resuscitated hBMSC was planted into the coated culture dish, a complete culture medium (a ratio of a NutriStem^{®} MSC XF basic culture medium to a NutriStem^{®} MSC XF additive is equal to 500ml:3ml) was added, culture was performed in a 5% CO₂ incubator at 37°C, and the culture medium was changed once every two days; and when the cell saturation reached about 80%, a complete culture medium containing ascorbic acid (50µg/mL) was used, the solution was changed once every two days in a dark place, the culture solution was discarded after ten days, washing was performed with DPBS for three times, hypotonic operation was performed with ultrapure water at 37°C for 10 min, the ultrapure water was removed, a cell lysis solution (3% TritonX-100, 2% lauryl sodium sulfate) was added to perform reaction for 5 min, washing was performed with DPBS, reaction was performed by using DNA enzyme (0.1mg/mL) at 37°C for 2 h after washing, and finally, washing was performed with DPBS for decellularization to obtain hBMSC-ECM.
(5) 5 guide fibers and the hBMSC-ECM were filled in the helical tube with the surface coated with the nanofiber membrane to obtain the bionic nerve graft, where the guide fibers are included in the tube wall of the helical stent.

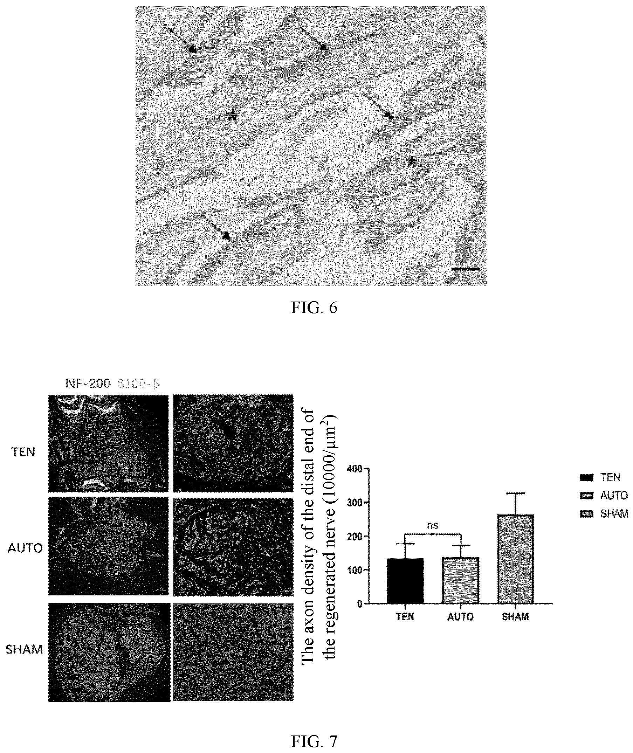

The hBMSC-ECM in the bionic nerve graft prepared in this embodiment contains bioactive components capable of promoting nerve regeneration, such as fibronectin, laminin and collagen I, and almost no DNA can be detected (as shown in FIG. 5). After acetylation of the helical stent semi-finished product, the acetylation degree is increased from 4.73% to 74.77%, and high in-vivo degradability is achieved (as shown in FIG. 6). The diameter of the electrostatic spinning nanofiber membrane with which the outer layer is coated is about 100 nm.

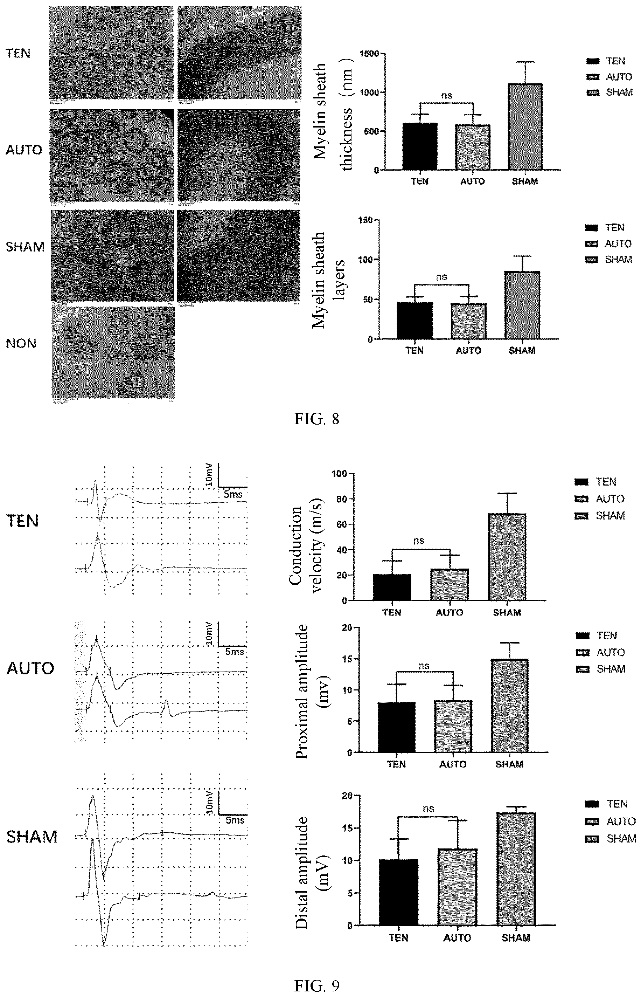

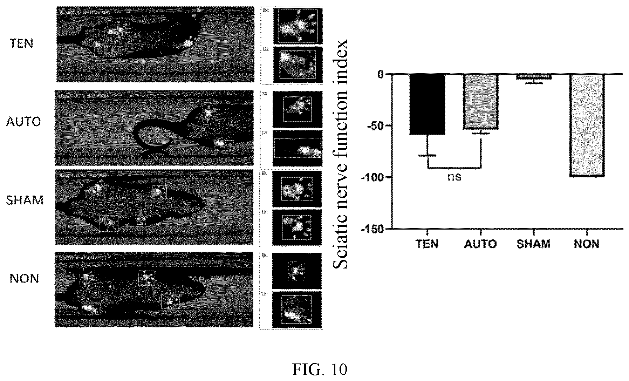

The bionic nerve graft prepared in this embodiment is used to repair the 10mm defect of the sciatic nerve of the rat. 8 weeks after the operation, the results of the immunofluorescence picture of the transverse section of the distal end of the regenerated nerve (as shown in FIG. 7) and the transmission electron microscope picture (as shown in FIG. 8) show that the bionic nerve graft has a good function of promoting nerve regeneration, and the effect of promoting nerve regeneration is equivalent to that of the autogenous nerve. 12 weeks after the operation, the function test (as shown in FIG. 9 and FIG. 10) shows that the bionic nerve has a good effect of repairing the injured nerve, and the repairing effect is similar to that of the autogenous nerve.

### Embodiment 3

(1) Chitosan was dissolved in an acetic acid solution of 2% and was stirred for 4 h until being completely dissolved to obtain a chitosan solution with the concentration of 7wt%; the chitosan solution was poured on a threaded inner core mold, where the inner mold has a length of 6cm, a diameter of 5mm, a thread pitch of 10cm and a line width of 5mm; the inner core mold with the chitosan solution was put into a NaOH solution of 20% for curing and shaping, was washed with water after being shaped, and was dried and demolded to obtain a helical stent semi-finished product; the helical stent semi-finished product was immersed into an acetic anhydride solution containing 5wt% methanol for acetylation for 6 h, then was fixed for 12 h at room temperature by a NaOH solution of 20%, and was washed and dried to obtain a helical stent.
(2) A proper amount of polyethylene oxide was dissolved into an acetic acid solution of 50%, and chitosan powder was added to prepare an electrostatic spinning solution, where the total concentration of the chitosan and the polyethylene oxide is 5wt% and the mass ratio of the chitosan to the polyethylene oxide is 100:5; the helical stent was placed in a rotatable tubular receiving apparatus, the electrostatic spinning solution was put into an electrostatic spinning machine injector, a voltage was set to 35kV, a flow velocity was set to 2ml/h, a receiving distance was set to 35cm, a rotating speed of the receiving apparatus was set to 150 rpm, electrostatic spinning was performed, and a surface of the helical stent was coated with one layer of nanofiber membrane to obtain a helical tube with a surface coated with the nanofiber membrane.
(3) The chitosan solution in step (1) was poured in a mold with a groove, where the shape of the groove is the same as that in Embodiment 1, a length of the groove is 1cm, a is 500 µm, b is 225 µm, and c is 50 µm; and the mold with the chitosan solution was put into a NaOH solution of 20% for curing and shaping, was washed with water after being shaped, and was dried and demolded to obtain guide fibers.
(4) A culture dish was coated with an MSC adherent reagent diluted 100 times by a DPBS solution; resuscitated hBMSC was planted into the coated culture dish, a complete culture medium (a ratio of a NutriStem^{®} MSC XF basic culture medium to a NutriStem^{®} MSC XF additive is equal to 500ml:3ml) was added, culture was performed in a 5% CO₂ incubator at 37°C, and the culture medium was changed once every two days; and when the cell saturation reached about 80%, a complete culture medium containing ascorbic acid (50µg/mL) was used, the solution was changed once every two days in a dark place, the culture solution was discarded after ten days, washing was performed with DPBS for three times, hypotonic operation was performed with ultrapure water at 37°C for 10 min, the ultrapure water was removed, a cell lysis solution (3% TritonX-100, 2% lauryl sodium sulfate) was added to perform reaction for 5 min, washing was performed with DPBS, reaction was performed by using DNA enzyme (0.1mg/mL) at 37°C for 2 h after washing, and finally, washing was performed with DPBS for decellularization to obtain hBMSC-ECM.
(5) 3 guide fibers and the hBMSC-ECM were filled in the helical tube with the surface coated with the nanofiber membrane to obtain the bionic nerve graft, where the guide fibers are included in the tube wall of the helical stent.

## Claims

1. A bionic nerve graft, comprising a helical stent, a nanofiber membrane, guide fibers and hBMSC-ECM, wherein a surface of the helical stent is coated with the nanofiber membrane; the guide fibers and the hBMSC-ECM are placed in a tube cavity of the helical stent; and the raw materials of the helical stent, the nanofiber membrane and the guide fibers are all chitosan.

2. The bionic nerve graft according to Claim 1, wherein the helical stent has an inner diameter of 1-6 mm, a length of 1-6 cm and a pitch of 0.5-5 mm.

3. The bionic nerve graft according to Claim 1, wherein the guide fibers have a length of 1-3 cm, and the number of the guide fibers is 3-28.

4. A preparation method of the bionic nerve graft according to any one of Claims 1-3, comprising the following steps:
using the chitosan as a raw material and preparing the helical stent by a mold pouring method;
using the chitosan as a raw material and coating the surface of the helical stent with one layer of nanofiber membrane to obtain a helical tube with a surface coated with the nanofiber membrane;
using the chitosan as a raw material and preparing the guide fibers by a template method; and
filling the guide fibers and the hBMSC-ECM in the helical tube with the surface coated with the nanofiber membrane to obtain the bionic nerve graft.

5. The preparation method of the bionic nerve graft according to Claim 4, wherein the step of preparing the helical stent comprises:
preparing a chitosan solution with a concentration of 4wt%-7wt%;
pouring the chitosan solution on a threaded inner core mold;
placing the inner core mold with the chitosan solution into a NaOH solution of 5-20% for curing and shaping, and then performing washing with water, drying and demolding to obtain a helical stent semi-finished product; and
immersing the helical stent semi-finished product into an acetic anhydride solution containing methanol of 5wt% to perform acetylation for 6 h, performing fixation for 12 h by using the NaOH solution of 5-20%, and washing and drying to obtain the helical stent.

6. The preparation method of the bionic nerve graft according to Claim 4, wherein the step of preparing the helical tube with the surface coated with the nanofiber membrane comprises:
preparing a blended solution of the chitosan and polyethylene oxide with the total concentration of 3wt%-5wt%, the mass ratio of the chitosan to the polyethylene oxide being 100:(5-20);
placing the blended solution of the chitosan and the polyethylene oxide into an electrostatic spinning apparatus, placing the helical stent on a tubular receiving apparatus, performing electrostatic spinning, and coating the surface of the helical stent with one layer of nanofiber membrane; and
placing the helical stent coated with the nanofiber membrane into a NaOH solution of 5-20% for curing and shaping, performing washing with water to remove the polyethylene oxide to obtain the helical tube with the surface coated with the nanofiber membrane.

7. The preparation method of the bionic nerve graft according to Claim 6, wherein setting parameters of the electrostatic spinning comprise: a voltage is 5-35 kV, a flow velocity is 0.1-2 mL/h, a receiving distance is 5-35 cm, and a rotating speed of the receiving apparatus is 5-150 rpm.

8. The preparation method of the bionic nerve graft according to Claim 4, wherein the step of preparing the guide fibers comprises:
preparing a chitosan solution with a concentration of 4wt%-7wt%;
pouring the chitosan solution in a mold with a groove; and
putting the mold with the chitosan solution into a NaOH solution of 5-20% for curing and shaping, and then performing washing with water, drying and demolding to obtain the guide fibers.

9. The preparation method of the bionic nerve graft according to Claim 8, wherein the groove of the mold comprises an inner groove and an outer groove which communicate with each other, the cross-sectional shape of the communicated inner groove and outer groove is shaped like an inverted Chinese character "Pin", a width of the outer groove is 100-500 µm, a height of the outer groove is 20-50 µm, a width of the inner groove is the same as the height of the outer groove, a height of the inner groove is 25-240 µm, the width of the outer groove is twice the sum of the height of the inner groove and the width of the inner groove, and the inner groove and the outer groove have the same length of 1-3 cm.

10. The preparation method of the bionic nerve graft according to Claim 4, wherein the step of preparing the hBMSC-ECM comprises:
coating a culture dish with an MSC adherent reagent diluted 100 times by a DPBS solution;
planting resuscitated hBMSC into the coated culture dish, adding a complete culture medium, performing culture in a 5% CO₂ incubator at 37°C, and changing the culture medium once every two days; and
when the cell saturation reaches 80%, using a complete culture medium containing ascorbic acid, changing the solution once every two days in a dark place, discarding the culture solution after ten days, performing washing with DPBS for three times, performing hypotonic operation with ultrapure water at 37°C for 10 min, removing the ultrapure water, adding a cell lysis solution to perform reaction for 5 min, performing washing with DPBS, performing reaction by using DNA enzyme at 37°C for 2 h after washing, and finally, performing washing with DPBS and performing storage at 4°C.

## Patentansprüche

1. Bionisches Nerventransplantat, umfassend einen spiralförmigen Stent, eine Nanofasermembran, Führungsfasern und hBMSC-ECM, wobei eine Oberfläche des spiralförmigen Stents mit der Nanofasermembran beschichtet ist; die Führungsfasern und das hBMSC-ECM in einem Hohlraum des spiralförmigen Stents angeordnet sind; und die Ausgangsmaterialien des spiralförmigen Stents, der Nanofasermembran und der Führungsfasern alle Chitosan sind.

2. Das bionische Nerventransplantat nach Anspruch 1, wobei der spiralförmige Stent einen Innendurchmesser von 1-6 mm, eine Länge von 1-6 cm und eine Steigung von 0,5-5 mm aufweist.

3. Das bionische Nerventransplantat nach Anspruch 1, wobei die Führungsfasern eine Länge von 1-3 cm aufweisen und die Anzahl der Führungsfasern 3-28 beträgt.

4. Verfahren zur Herstellung des bionischen Nerventransplantats nach einem der Ansprüche 1 bis 3, umfassend die folgenden Schritte:
Verwendung von Chitosan als Ausgangsmaterial und Herstellung des spiralförmigen Stents durch ein Formgussverfahren;
Verwendung von Chitosan als Ausgangsmaterial und Beschichtung der Oberfläche des spiralförmigen Stents mit einer Schicht aus einer Nanofasermembran, um ein spiralförmiges Rohr zu erhalten, dessen Oberfläche mit der Nanofasermembran beschichtet ist;
Verwendung von Chitosan als Ausgangsmaterial und Herstellung der Führungsfasern mittels eines Schablonenverfahrens; und
das Einfüllen der Führungsfasern und des hBMSC-ECM in das spiralförmige Rohr, dessen Oberfläche mit der Nanofasermembran beschichtet ist, um das bionische Nerventransplantat zu erhalten.

5. Verfahren zur Herstellung des bionischen Nerventransplantats gemäß Anspruch 4, wobei der Schritt der Herstellung des spiralförmigen Stents umfasst:
Herstellung einer Chitosan-Lösung mit einer Konzentration von 4 Gew.-% bis 7 Gew.-%;
das Gießen der Chitosan-Lösung auf eine mit Gewinde versehene Innenkernform;
das Einlegen der Innenkernform mit der Chitosanlösung in eine 5-20-prozentige NaOH-Lösung zum Aushärten und Formen, gefolgt von Waschen mit Wasser, Trocknen und Entformen, um ein Halbzeug des spiralförmigen Stents zu erhalten; und
das Halbzeug des spiralförmigen Stents in eine 5 Gew.-%ige Essigsäureanhydrid-Lösung, die Methanol enthält, eintauchen, um eine Acetylierung über 6 Stunden durchzuführen, eine Fixierung über 12 Stunden unter Verwendung der 5-20 %igen NaOH-Lösung vornehmen sowie das Produkt waschen und trocknen, um den spiralförmigen Stent zu erhalten.

6. Verfahren zur Herstellung des bionischen Nerventransplantats gemäß Anspruch 4, wobei der Schritt der Herstellung des spiralförmigen Rohrs mit der mit der Nanofasermembran beschichteten Oberfläche umfasst:
Herstellung einer Mischlösung aus Chitosan und Polyethylenoxid mit einer Gesamtkonzentration von 3 Gew.-% bis 5 Gew.-%, wobei das Massenverhältnis von Chitosan zu Polyethylenoxid 100:(5-20) beträgt;
das Einbringen der Mischlösung aus Chitosan und Polyethylenoxid in eine elektrostatische Spinnvorrichtung, das Aufbringen des spiralförmigen Stents auf eine röhrenförmige Aufnahmegerät, das Durchführen des elektrostatischen Spinnens und das Beschichten der Oberfläche des spiralförmigen Stents mit einer Schicht aus Nanofasermembran; und
Einlegen des mit der Nanofasermembran beschichteten spiralförmigen Stents in eine 5-20-prozentige NaOH-Lösung zum Aushärten und Formen, Durchführen einer Waschung mit Wasser, um das Polyethylenoxid zu entfernen, um so das spiralförmige Rohr mit der mit der Nanofasermembran beschichteten Oberfläche zu erhalten.

7. Verfahren zur Herstellung des bionischen Nerventransplantats nach Anspruch 6, wobei die Einstellparameter für das elektrostatische Spinnen Folgendes umfassen: eine Spannung von 5-35 kV, eine Durchflussgeschwindigkeit von 0,1-2 ml/h, einen Aufnahmabstand von 5-35 cm und eine Drehzahl der Aufnahmevorrichtung von 5-150 U/min.

8. Verfahren zur Herstellung des bionischen Nerventransplantats nach Anspruch 4, wobei der Schritt der Herstellung der Führungsfasern umfasst:
Herstellung einer Chitosan-Lösung mit einer Konzentration von 4 Gew.-% bis 7 Gew.-%;
das Eingießen der Chitosan-Lösung in eine Form mit einer Rille; und
das Einlegen der Form mit der Chitosanlösung in eine 5-20-prozentige NaOH-Lösung zum Aushärten und Formen, gefolgt von Waschen mit Wasser, Trocknen und Entformen, um die Führungsfasern zu erhalten.

9. Verfahren zur Herstellung des bionischen Nerventransplantats gemäß Anspruch 8, wobei die Rille der Form eine innere Rille und eine äußere Rille umfasst, die miteinander in Verbindung stehen, wobei die Querschnittsform der miteinander verbundenen inneren und äußeren Rille die Form eines umgekehrten chinesischen Schriftzeichens "Pin" aufweist, die Breite der äußeren Rille 100-500 µm beträgt, die Höhe der äußeren Nut 20-50 µm beträgt, die Breite der inneren Nut der Höhe der äußeren Nut entspricht, die Höhe der inneren Nut 25-240 µm beträgt, die Breite der äußeren Nut das Doppelte der Summe aus der Höhe der inneren Nut und der Breite der inneren Nut beträgt und die innere Nut und die äußere Nut die gleiche Länge von 1-3 cm aufweisen.

10. Verfahren zur Herstellung des bionischen Nerventransplantats gemäß Anspruch 4, wobei der Schritt der Herstellung des hBMSC-ECM umfasst:
das Beschichten einer Kulturschale mit einem MSC-Adhäsionsreagenz, das 100-fach mit einer DPBS-Lösung verdünnt wurde;
das Einbringen von reanimierten hBMSC in die beschichtete Kulturplatte, das Zugabe eines vollständigen Kulturmediums, das Durchführen der Kultivierung in einem Inkubator mit 5 % _{CO₂}bei 37 °C sowie das Wechseln des Kulturmediums alle zwei Tage; und
wenn die Zellsättigung 80 % erreicht, Verwendung eines vollständigen Kulturmediums, das Ascorbinsäure enthält, Wechsel der Lösung alle zwei Tage an einem dunklen Ort, Entsorgung der Kulturlösung nach zehn Tagen, dreimaliges Waschen mit DPBS, Durchführung einer hypotonischen Behandlung mit ultrareinem Wasser bei 37 °C , das Reinstwasser entfernen, eine Zelllyselösung hinzufügen und 5 Minuten lang reagieren lassen, mit DPBS waschen, nach dem Waschen 2 Stunden lang bei 37 °C eine Reaktion mit einem DNA-Enzym durchführen und schließlich mit DPBS waschen und bei 4 °C lagern.

## Revendications

1. Greffe nerveuse bionique, comprenant un stent hélicoïdal, une membrane en nanofibres, des fibres de guidage et de l'hBMSC-ECM, dans laquelle une surface du stent hélicoïdal est recouverte de la membrane en nanofibres ; les fibres de guidage et l'hBMSC-ECM sont placés dans une cavité tubulaire du stent hélicoïdal ; et les matières premières du stent hélicoïdal, de la membrane en nanofibres et des fibres de guidage sont toutes du chitosane.

2. Greffe nerveuse bionique selon la revendication 1, dans laquelle le stent hélicoïdal présente un diamètre intérieur de 1 à 6 mm, une longueur de 1 à 6 cm et un pas de 0,5 à 5 mm.

3. Greffe nerveuse bionique selon la revendication 1, dans laquelle les fibres de guidage ont une longueur comprise entre 1 et 3 cm, et leur nombre est compris entre 3 et 28.

4. Procédé de fabrication de la greffe nerveuse bionique selon l'une quelconque des revendications 1 à 3, comprenant les étapes suivantes :
utiliser le chitosane comme matière première et préparer le stent hélicoïdal par un procédé de coulée en moule ;
utiliser le chitosane comme matière première et recouvrir la surface du stent hélicoïdal d'une couche de membrane en nanofibres afin d'obtenir un tube hélicoïdal dont la surface est recouverte de ladite membrane en nanofibres ;
utiliser le chitosane comme matière première et préparer les fibres de guidage par un procédé de moulage ; et
remplir le tube hélicoïdal, dont la surface est recouverte de la membrane en nanofibres, avec les fibres de guidage et l'hBMSC-ECM afin d'obtenir la greffe nerveuse bionique.

5. Procédé de préparation de la greffe nerveuse bionique selon la revendication 4, dans lequel l'étape de préparation du stent hélicoïdal comprend :
la préparation d'une solution de chitosane à une concentration comprise entre 4 % et 7 % en poids ;
verser la solution de chitosane sur un moule à noyau interne fileté ;
placer le moule à noyau interne contenant la solution de chitosane dans une solution de NaOH à 5-20 % pour le durcissement et le formage, puis procéder au lavage à l'eau, au séchage et au démoulage afin d'obtenir un produit semi-fini de stent hélicoïdal ; et
plonger le produit semi-fini de stent hélicoïdal dans une solution d'anhydride acétique contenant 5 % en poids de méthanol pour effectuer une acétylation pendant 6 h, procéder à une fixation pendant 12 h à l'aide de la solution de NaOH à 5-20 %, puis effectuer un lavage et un séchage pour obtenir le stent hélicoïdal.

6. Procédé de préparation de la greffe nerveuse bionique selon la revendication 4, dans lequel l'étape de préparation du tube hélicoïdal dont la surface est recouverte d'une membrane de nanofibres comprend :
la préparation d'une solution mélangée de chitosane et de poly(oxyde d'éthylène) à une concentration totale comprise entre 3 % et 5 % en masse, le rapport massique entre le chitosane et le poly(oxyde d'éthylène) étant de 100 : (5 à 20) ;
introduire la solution mélangée de chitosane et de poly(oxyde d'éthylène) dans un appareil de filage électrostatique, placer le stent hélicoïdal sur un dispositif de réception tubulaire, effectuer le filage électrostatique et recouvrir la surface du stent hélicoïdal d'une couche de membrane en nanofibres ; et
placer le stent hélicoïdal recouvert de la membrane de nanofibres dans une solution de NaOH à 5-20 % pour le durcissement et la mise en forme, effectuer un lavage à l'eau pour éliminer le poly(oxyde d'éthylène) afin d'obtenir le tube hélicoïdal dont la surface est recouverte de la membrane de nanofibres.

7. Procédé de préparation de la greffe nerveuse bionique selon la revendication 6, dans lequel les paramètres de réglage du filage électrostatique comprennent : une tension de 5 à 35 kV, une vitesse d'écoulement de 0,1 à 2 ml/h, une distance de réception de 5 à 35 cm, et une vitesse de rotation du dispositif de réception de 5 à 150 tr/min.

8. Procédé de préparation de la greffe nerveuse bionique selon la revendication 4, dans lequel l'étape de préparation des fibres de guidage comprend :
la préparation d'une solution de chitosane à une concentration comprise entre 4 % et 7 % en poids ;
verser la solution de chitosane dans un moule muni d'une rainure ; et
placer le moule contenant la solution de chitosane dans une solution de NaOH à 5-20 % pour le durcissement et le formage, puis procéder à un lavage à l'eau, à un séchage et au démoulage pour obtenir les fibres de guidage.

9. Procédé de préparation de la greffe nerveuse bionique selon la revendication 8, dans lequel la rainure du moule comprend une rainure intérieure et une rainure extérieure qui communiquent entre elles, la forme en coupe transversale de la rainure intérieure et de la rainure extérieure communicantes a la forme d'un caractère chinois « Pin » inversé, la largeur de la rainure extérieure est comprise entre 100 et 500 µm, la hauteur de la rainure extérieure est comprise entre 20 et 50 µm, la largeur de la rainure intérieure est égale à la hauteur de la rainure extérieure, la hauteur de la rainure intérieure est comprise entre 25 et 240 µm, la largeur de la rainure extérieure est égale au double de la somme de la hauteur de la rainure intérieure et de la largeur de la rainure intérieure, et la rainure intérieure et la rainure extérieure ont la même longueur, comprise entre 1 et 3 cm.

10. Procédé de préparation de la greffe nerveuse bionique selon la revendication 4, dans lequel l'étape de préparation de l'hBMSC-ECM comprend :
le revêtement d'une boîte de culture avec un réactif d'adhérence des MSC dilué 100 fois dans une solution de DPBS ;
la mise en culture de hBMSC réanimées dans la boîte de culture enduite, l'ajout d'un milieu de culture complet, la culture dans un incubateur à 5 % _{de CO₂}à 37 °C, et le renouvellement du milieu de culture tous les deux jours ; et
lorsque la saturation cellulaire atteint 80 %, utiliser un milieu de culture complet contenant de l'acide ascorbique, changer la solution tous les deux jours à l'abri de la lumière, jeter la solution de culture au bout de dix jours, effectuer trois lavages avec du DPBS, réaliser un traitement hypotonique avec de l'eau ultra-pure à 37 °C pendant 10 minutes, éliminer l'eau ultra-pure, ajouter une solution de lyse cellulaire pour effectuer la réaction pendant 5 minutes, procéder à un lavage avec du DPBS, effectuer la réaction à l'aide d'une enzyme d'ADN à 37 °C pendant 2 heures après le lavage, puis enfin, procéder à un lavage avec du DPBS et conserver le tout à 4 °C.
